# EUROPÄISCHE PATENTANMELDUNG

(11) **EP 1 859 776 A2**
(43) Veröffentlichungstag der Anmeldung: **28.11.2007**
(21) Anmeldenummer: 07108532.8
(22) Anmeldetag: 21.05.2007
(51) Int. Cl.: A61K 8/35, A61K 8/37, A61K 8/44, A61K 8/49, A61Q 17/04

(54) **Kosmetisches Sonnenschutzmittel**

(30) Priorität: 22.05.2006 DE 102006024930
(71) Anmelder: Coty Prestige Lancaster Group GmbH, 55116 Mainz (DE)
(72) Erfinder: Golz-Berner, Karin, 98000, Monaco (MC); Zastrow, Leonhard, 98000, Monaco (MC); Bernini, Dorothée, 98000, Monaco (MC)
(74) Vertreter: Ziebig, Marlene

(57) **Zusammenfassung**

Die Erfindung betrifft neue Sonnenschutzmittel mit hohen Sonnenschutzfaktoren. Aufgabe der Erfindung ist es, den Gehalt an UV-Filtersubstanzen bei hohen Sonnenschutzfaktoren deutlich abzusenken und Hautirritationen zu verringern. Das Sonnenschutzmittel enthält 11,5-23 % organische UVA- und UVB-Sonnenschutzfilter, 0,4-1,2 % unter UV-Licht fluoreszierende Substanzen und übliche kosmetische Trägerstoffe, wobei das Mittel einen Sonnenschutzfaktor SPF im Bereich von SPF 12 bis SPF 35 hat.

## Beschreibung

Die Erfindung betrifft neue Sonnenschutzmittel mit hohen Sonnenschutzfaktoren.

Infolge zunehmenden Bewusstseins vieler Menschen, ihre Haut vor den schädlichen Einflüssen durch ultraviolette Strahlung zu schützen und doch eine attraktive Bräunung der Haut zu erhalten, sind in den letzten Jahren neue Sonnenschutzformulierungen mit immer höheren Sonnenschutzfaktoren (SPF = Sun Protection Factor) entwickelt worden. Da die Anzahl an zugelassenen Filtersubstanzen für Sonnenschutzformulierungen relativ gering ist, und bestimmte Höchstmengen für einzelne Substanzen festgelegt sind, meist nicht mehr als 10 %, geht die Tendenz mehr und mehr zu Kombinationen verschiedener Substanzen, um gerade die hohen SPF-Werte zu erreichen. Mit hohen Substanzkonzentrationen innerhalb einer Formulierung wächst auch das Risiko von Hautirritationen. Wenn eine Sonnenschutzformulierung mit einem SPF von 23 üblicherweise zirka 18 % organische Filtersubstanzen enthält oder eine mit SPF 30 zirka 28 %, so ist dies nicht immer für jede Haut verträglich, und es stellt auch einen erheblichen Kostenfaktor dar.

Der Erfindung liegt die Aufgabe zugrunde, Sonnenschutzmittel zu entwickeln, deren Gehalt an UV-Filtersubstanzen bei hohen Sonnenschutzfaktoren deutlich geringer ist als bisher.

Eine weitere Aufgabe der Erfindung besteht darin, die Gefahr von Hautirritationen durch Verringerung der absoluten Menge an organischen UV-Filtersubstanzen in Sonnenschutzmittel mit hohen SPF's zu vermindern.

Erfindungsgemäß ist das kosmetische Sonnenschutzmittel gekennzeichnet durch einen Gehalt an 11,5 bis 23 % organische UVA- und UVB-Sonnenschutzfilter, 0,4-1,2 % unter UV-Licht fluoreszierende Substanzen und den Rest bis 100 % übliche kosmetische Trägerstoffe, Hilfsstoffe, Wirkstoffe oder Gemische davon, wobei das kosmetische Mittel einen Sonnenschutzfaktor SPF im Bereich von SPF 12 bis SPF 35 hat und alle Prozentangaben auf das Gesamtgewicht des Mittels bezogen sind.

Es wurde gefunden, dass der Zusatz geringer Mengen von weniger als 2 Gew.-%, bevorzugt 0,6 - 1,1 Gew.-% von Verbindungen, die zusammen unter UV-Licht fluoreszieren und zugleich kosmetisch annehmbar sind, eine deutliche Absenkung des Gehaltes an Sonnenschutzfiltern, insbesondere organischen Sonnenschutzfiltern, ermöglicht. Ohne an eine Theorie gebunden zu sein, wird vermutet, dass die UV-Filtersubstanzen UV-Photonen absorbieren, die dabei entstehende Energie auf die fluoreszierenden Substanzen übertragen und diese zur Fluoreszenz anregen. Die Umwandlung von Wärmeenergie in abgestrahltes, sichtbares Fluoreszenzlicht könnte das Entstehen von freien Radikalen vermeiden, die üblicherweise in Wechselbeziehung mit den antioxidativen Schutzsystemen der Haut zur Erythembildung führen würden. Somit würde weniger UV-Licht in Wärme und damit in freie Radikale umgewandelt werden, und mit niedrigerer Konzentration an Sonnenschutzfiltern können gleiche oder bessere Wirkungen, d.h. höhere SPF's als zuvor erzielt werden.

Die auftretende Fluoreszenz ist eine optische Fluoreszenz, bei der die von der fluoreszierenden Verbindung aufgewandte Energie in Form von Photonen innerhalb von Nanosekunden als Lichtenergie abgestrahlt wird. Da durch sog. Quencher die Fluoreszenz vermindert wird, ist die Gegenwart von Sauerstoff in der Emulsion nicht bevorzugt. "Unter UV-Licht fluoreszierende Substanz" bedeutet im Sinne der Erfindung, dass diese Substanz jedenfalls in Anwesenheit der UVA- und UVB-Sonnenschutzfilter Fluoreszenz zeigt, aber auch alleine fluoreszieren kann.

Der Effekt der Verringerung sonst üblicher Mengen an Sonnenschutzfiltern bei gleichem SPF wurde durch eine Studie erhärtet und ist in den Vergleichsbeispielen näher beschrieben.

Vorzugsweise sind die unter den obigen Bedingungen und unter UV-Licht fluoreszierenden Substanzen weiße oder farblose Substanzen.

Die unter UV-Licht fluoreszierenden Substanzen sind bevorzugt aus der Gruppe ausgewählt, bestehend aus Ethylendiamintetraessigsäure, dem Dinatriumsalz der Ethylendiamintetraessigsäure, Trinatriumsalz von EDTA, Tetranatriumsalz von EDTA, Tryptophan, Phenylalanin und Tyrosin und Gemischen davon. Es können jedoch auch andere Substanzen eingesetzt werden wie z.B. Oxide von Seltenerdmetallen, sofern sie kosmetisch annehmbar sind.

Vorzugsweise ist die unter UV-Licht fluoreszierende Substanz das Dinatriumsalz von Ethylendiamintetraessigsäure oder ein Gemisch davon mit EDTA.

Die erfindungsgemäßen Sonnenschutzmittel können weitere kosmetisch übliche Wirkstoffe enthalten. Dazu gehören beispielsweise Radikalfänger, Feuchthaltemittel, Vitamine, Erweichungsmittel, Enzyme, pflanzliche Wirkstoffe, Polymere, Antioxidationsmittel, entzündungswidrige natürliche Wirkstoffe.

Als Radikalfänger sind speziell bevorzugt Vitamin A, Vitamin E, Peptide, Flavone, Flavonole, sowie bestimmte Pflanzenextrakte.

Eine vorteilhafte Ausführungsform der Erfindung besteht darin, den Anteil an Radikalfängern auf ein Minimum abzusenken und vorzugsweise weniger als 1,5 Gew.-%, insbesondere weniger als 1,2 Gew.-% und speziell etwa nur 0,5 - 0,1 Gew.-% an radikalfangenden Substanzen einzusetzen. Dies steht im Zusammenhang mit dem obigen vermutlichen Wirkungsmechanismus, wonach der Gehalt an Sonnenschutzfiltern reduziert ist und die unter UV-Licht fluoreszierende Substanz einen erheblichen Teil der sonst freien Radikale vermeidet.

Zu Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; einige Aminosäuren und Derivate davon; Carotinoide und Carotine, wie z.B α-Carotin, β-Carotin; Harnsäure und Derivate davon; α-Hydroxysäuren wie Citronensäure, Milchsäure, Apfelsäure; Pflanzenextrakte sowie Gemische von diesen Substanzen.

Bevorzugte Antioxidationsmittel bzw. Radikalfänger sind Pflanzenextrakte, insbesondere solche, die aus der Gruppe ausgewählt sind, bestehend aus Angelica archangelica, Arnica montana, Camellia sinensis, Cupressus semper, Coffee arabica, Polygonatum multiflorum, Pongamia pinnata, Rosmarinus officinalis, Evernia fufuracea, Evernia prunastri, Aventa sativa und Gemische davon. Ein besonders vorteilhafter Pflanzenextrakt ist ein solcher aus getrockneten Extrakten von Angelica archangelica, Camellia sinensis, Coffee arabica und Pongamia pinnata in einem einwertigen Alkohol.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Feuchthaltemittel sind beispielsweise Glycerin, Butylenglycol, Propylenglycol und Gemische davon.

Das erfindungsgemäße Präparat enthält weiterhin kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, weichmachende Substanzen, feuchthaltende Substanzen, Duftstoffe, Alkohole, Polyole, Elektrolyte, Gelbildner, polare und unpolare Öle, Polymere, Copolymere, Emulgatoren, Wachse, Stabilisatoren.

Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titanoxid, Glimmer, Kaolin, manganhaltige Tone, Calciumcarbonat, Talkum, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken. Ebenso können andere organische Farbstoffe, die in der Kosmetik üblich sind, als Färbemittel eingesetzt werden.

Als oberflächenaktive Mittel können anionische, amphotere, nichtionische oder kationische oberflächenaktive Mittel oder Gemische davon eingesetzt werden. Besonders bevorzugt sind kationische Polymere oder ein Gemisch von anionischen und amphoteren oberflächenaktiven Mitteln. Ein Gemisch von anionischen mit amphoteren oberflächenaktiven Mitteln ist bevorzugt.

Die für die Erfindung eingesetzten Öle können übliche kosmetische Öle sein, wie synthetische Öle, Pflanzenöle und Siliconöle. Bevorzugt sind Siliconöle, wie beispielsweise cyclische Siliconöle, z.B. Cyclotetrasiloxane, Cyclopentasiloxane und Cyclohexasiloxane oder Gemische davon. Auch andere Siliconöle wie Dimethicone können eingesetzt werden.

Polyole sind ebenfalls mögliche Bestandteile des erfindungsgemäßen Gels. Dies sind z.B Propylenglycol, Dipropylenglycol, Ethylenglycol, Isoprenglycol, Glycerin, Butylenglycole, Sorbitol und Gemische davon.

Als Ester oder Ether sind zum Beispiel geeignet, wie Diisopropylsebacat, Behenylakohol, Glycerylstearat, Dibytyladipat, Cetearylalkohol, Dicaprylylcarbonat usw.

Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Die erfindungsgemäßen Mittel enthalten UVA- und UVB-Sonnenschutzfilter, wobei vorteilhaft das Verhältnis zwischen UVB- und UVA-Sonnenschutzfiltern im Bereich von 1:1,1-1,8, vorzugsweise 1:1,15-1,3 liegt. Zu diesen Filtern gehören 4-Aminobenzoesäure-Derivate, Ester der Zimtsäure, Benzophenon-Derivate, Triazine, Phenole, Salicylate, Triazone und 3-Benzylidencampher-Derivate sowie Sulfonsäurederivate des Benzophenons.

Bevorzugte UV-Filter sind Butyl-Methoxybenzoylmethane, Octyl Methoxycinnamate, Octyl Salicylate, 4-Methylbenzylidene Camphor, Homosalate, Octyl Dimethyl PABA, Butyl Methoxybenzoylmethane. *

Besonders bevorzugt sind Butyl Methoxydibenzoylmethane, 4-Methylbenzylidene Camphor, Ethylhexyl Methoxycinnamate, Isoamyl-p-Methoxycinnamate, Ethylhexyl Triazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine, Diethylamino Hydroxybenzoyl Hexylbenzoate.

Es wird bevorzugt, dass der Anteil der organischen UVB-Sonnenschutzfilter im Bereich von 6-13 Gew.-% liegt und der Anteil der organischen UVA-Sonnenschutzfilter im Bereich von 5-11 Gew.-% liegt. Das Verhältnis von UVA- zu UVB-Sonnenschutzfiltern liegt bevorzugt im Bereich von 1:1,1 bis 1,3. Dies ist für das Erreichen des Fluoreszenzeffektes wichtig.

Besonders bevorzugt ist ein Sonnenschutzmittel, wenn es als Sonnenschutzfilter Diethylamino Hydroxybenzoyl Hexyl Benzoate & Ethylhexyl Methoxycinnamate, Ethylhexyl Triazone, Ethylhexyl Salicylate, Bis-Ethylhexyloxyphenol Methoxyphenyl Triazine, Butyl Methoxydibenzoylmethane, Methylene Bis-Benzotriazolyl & Tetramethylbutylphenol und Ethylhexyl Methoxycinnamate und wenigstens 0,6 % EDTA oder ein Salz von EDTA oder ein Gemisch davon enthält.

Gegenstand der Erfindung ist auch die Verwendung von unter UV-Licht fluoreszierenden Verbindungen in einer Menge von 0,4 - 1,2 Gew.-%, gemeinsam mit UV-Sonnenschutzfiltern, vorzugsweise einem Gemisch von mehreren organischen UV-B-Filtern und einem oder mehreren organischen UV-A-Filtern in Sonnenschutzmitteln mit einem Sonnenschutzfaktor von SPF 12 bis SPF 35, wobei der Gehalt an UV-Filtern im Bereich von 11,5 bis 23 Gew.-% liegt, um den Gehalt an UV-Filtern, der normalerweise für diese Sonnenschutzfaktoren erforderlich wäre, zu verringern.

Ein weiterer Gegenstand der Erfindung ist die Verwendung von unter UV-Licht fluoreszierenden Verbindungen in einer Menge von 0,4 - 1,2 Gew.-%, gemeinsam mit UV-Sonnenschutzfiltern, vorzugsweise einem Gemisch von mehreren organischen UV-B-Filtern und einem oder mehreren organischen UV-A-Filtern, in Sonnenschutzmitteln mit einem Sonnenschutzfaktor von SPF 12 bis SPF 35, wobei der Gehalt an UV-Filtern im Bereich von 11,5 bis 23 Gew.-% liegt, um durch Konzentrationen von größer als 21 % an organischen UV-Sonnenschutzfiltern hervorgerufene Hautirritationen zu verringern oder zu vermeiden.

Die Messung des SPF erfolgt nach der bekannten COLIPA-Methode in der aktuellen Fassung (CTFA Südafrika, CTFA Europa, JCIA Japan - 2003) bei jeweils 15 Probanden.

Der Einsatz des erfindungsgemäßen kosmetischen Präparates kann z.B. erfolgen in Sonnencremes, Sonnengelen, Tagescremes, Sonnensprays, Sonnenmilch, Sonnenlotionen, Lippengelen, Augencremes, Antifaltencremes, Antifaltengelen, Antialterungslotionen.

Die Erfindung soll nachstehend durch Beispiele näher erläutert werden. Alle Angaben erfolgen in Gewichtsprozent, sofern nichts anderes angegeben ist.

### Beispiel 1 Anti-Alterungscreme SPF 15

| **Phase A** | |
|---|---|
| Wasser | q.s. ad 100 |
| Caffeine | 0,5 |
| Sodium Polyacrylate | 0,6 |
| Disodium EDTA | 0,6 |
| Propylene Glycol | 3,0 |
| Diethylamino Hydroxybenzoyl Hexyl Benzoate & Ethylhexyl Methoxycinnamate | 2,0 |

| **Phase B** | |
|---|---|
| Ethylhexyl Triazone | 1,5 |
| Bis-Ethylhexyloxyphenol Methoxyphenyl Triazine | 1,0 |
| Butyl Methoxydibenzoylmethane | 3,0 |
| Ethylhexyl Methoxycinnamate BHT | 2,0 |
| Diisopropyl Sebacate | 8,0 |
| Dibutyl Adipate | 3,0 |
| Behenyl Alcohol & Glyeryl Stearate & Glyceryl Stearate Citrate & Sodium Dicocoylethylenediamine PEG-15 Sulfate | 6,0 |
| Cetearyl Alcohol | 1,0 |
| Cyclisches Siliconöl | 4,5 |
| Talkum | 2,0 |
| Methylene Bis-Benzotriazolyl & Tetramethylbutylphenol | 3,0 |

| **Phase C** | |
|---|---|
| Alcohol | 3,0 |
| Hydrolyzed Viola Tricolor Extract | 0,1 |
| Biotanning® | 0,25 |
| Sodium Lactate Methylsilanol & Salicylic Acid | 0,1 |
| RPF-Komplex* | 0,15 |
| Parfum | 0,8 |
| Phenoxyethanol | 0,6 |

| | |
|---|---|
| *Bedeutung: Extrakt von Angelica Archangelica Root & Camellia Sinensis Leaf & Pongamia Pinnata & Coffea Arabica zu gleichen Teilen in Alkohol - gemäß WO2004/105706 Beispiel 1. | |

Die Phasen A und B wurden separat bei 70 °C hergestellt und unter Rühren zusammengegeben. Nach dem Abkühlen auf etwa 35 °C wurde die ebenfalls separat hergestellte Phase C unter Rühren hinzugegeben. UV-Filter gesamt: 12,5 Gew.-%.

Vergleichsbeispiel 1a **Anti-Alterungscreme SPF 15**

| **Phase A** | |
|---|---|
| Wasser | q.s. ad 100 |
| Caffeine | 0,7 |
| Sodium Polyacrylate | 0,6 |
| Disodium EDTA | 0,1 |
| Propylene Glycol | 3,0 |
| Diethylamino Hydroxybenzoyl Hexyl Benzoate & Ethylhexyl Methoxycinnamate | 2,0 |

| **Phase B** | |
|---|---|
| Ethylhexyl Triazone | 2,0 |
| Bis-Ethylhexyloxyphenol Methoxyphenyl Triazine | 1,0 |
| Butyl Methoxydibenzoylmethane | 4,0 |
| Ethylhexyl Methoxycinnamate BHT | 3,0 |
| Diisopropyl Sebacate | 8,0 |
| Dibutyl Adipate | 3,0 |
| Behenyl Alcohol & Glyeryl Stearate & Glyceryl Stearate Citrate & Sodium Dicocoylethylenediamine PEG-15 Sulfate | 6,0 |
| Cetearyl Alcohol | 1,0 |
| Cyclisches Siliconöl | 4,5 |
| Talkum | 2,0 |
| Methylene Bis-Benzotriazolyl & Tetramethylbutylphenol | 5,0 |

| **Phase C** | |
|---|---|
| Alcohol | 3,0 |
| Hydrolyzed Viola Tricolor Extract | 0,3 |
| Biotanning® | 0,25 |
| Sodium Lactate Methylsilanol & Salicylic Acid | 0,1 |
| RPF-Komplex* | 0,55 |
| Parfum | 0,8 |
| Phenoxyethanol | 0,6 |

Die Verfahrensweise entsprach der von Beispiel 1.
UV-Filter gesamt: 17 Gew.-%.

### Beispiel 2 Anti-Alterungscreme SPF 30

| **Phase A** | |
|---|---|
| Wasser | q.s. ad 100 |
| Caffeine | 0,3 |
| Sodium Polyacrylate | 0,8 |
| Disodium EDTA | 0,4 |
| Propylene Glycol | 3,0 |
| Ethylhexyl Methoxycinnamate BHT | 5,0 |

| **Phase B** | |
|---|---|
| Ethylhexyl Triazone | 2,0 |
| Ethylhexyl Salicylate | 5,0 |
| Bis-Ethylhexyloxyphenol Methoxyphenyl Triazine | 2,0 |
| Butyl Methoxydibenzoylmethane | 3,0 |
| Dicaprylyl Carbonate | 4,0 |
| Dibutyl Adipate | 4,5 |
| Behenyl Alcohol & Glyeryl Stearate & Glyceryl Stearate Citrate & Sodium Dicocoylethylenediamine PEG-15 Sulfate | 7,0 |
| Cetearyl Alcohol | 3,0 |
| Cyclisches Siliconöl | 5,0 |
| Dimethicone & Cyclotetrasiloxane | 3,0 |
| Methylmethacrylate Crosspolymer | 2,0 |
| Methylene Bis-Benzotriazolyl & | |
| Tetramethylbutylphenol | 5,0 |

| **Phase C** | |
|---|---|
| Hydrolyzed Viola Tricolor Extract | 0,1 |
| Phototan® | 0,25 |
| Sodium Lactate Methylsilanol & | |
| Salicylic Acid | 0,5 |
| RPF-Komplex* | 0,4 |
| Parfum | 0,8 |
| Phenoxyethanol | 0,9 |

Die Verfahrensweise entsprach der von Beispiel 1.
UV-Filter gesamt: 22 Gew.-%.

### Vergleichsbeispiel 2a Anti-Alterungscreme SPF 30

| **Phase A** | |
|---|---|
| Wasser | q.s. ad 100 |
| Caffeine | 0,4 |
| Sodium Polyacrylate | 0,8 |
| Propylene Glycol | 3,0 |
| Ethylhexyl Methoxycinnamate BHT | 6,0 |

| **Phase B** | |
|---|---|
| Ethylhexyl Triazone | 2,0 |
| Ethylhexyl Salicylate | 7,0 |
| Bis-Ethylhexyloxyphenol Methoxyphenyl Triazine | 5,0 |
| Butyl Methoxydibenzoylmethane | 5,0 |
| Dicaprylyl Carbonate | 4, 0 |
| Dibutyl Adipate | 4,5 |
| Behenyl Alcohol & Glyeryl Stearate & Glyceryl Stearate Citrate & Sodium Dicocoylethylenediamine PEG-15 Sulfate | 7,0 |
| Cetearyl Alcohol | 3,0 |
| Cyclisches Siliconöl | 5,0 |
| Dimethicone & Cyclotetrasiloxane | 3,0 |
| Methylmethacrylate Crosspolymer | 2,0 |
| Methylene Bis-Benzotriazolyl & Tetramethylbutylphenol | 8,0 |

| **Phase C** | |
|---|---|
| Hydrolyzed Viola Tricolor Extract | 0,1 |
| Phototan® | 0,45 |
| Sodium Lactate Methylsilanol & Salicylic Acid | 0,5 |
| RPF-Komplex* | 0,8 |
| Parfum | 0,8 |
| Phenoxyethanol | 0,9 |

Die Verfahrensweise entsprach der von Beispiel 1.
UV-Filter gesamt: 33 Gew.-%.

### Beispiel 3 Anti-Alterungscreme SPF 20

| **Phase A** | |
|---|---|
| Wasser | q.s. ad 100 |
| Caffeine | 0,45 |
| Sodium Polyacrylate | 0,6 |
| Disodium EDTA / EDTA (60:40) | 0,7 |
| Propylene Glycol Diethylamino Hydroxybenzoyl Hexyl Benzoate & | 3,5 |
| Ethylhexyl Methoxycinnamate | 3,0 |

| **Phase B** | |
|---|---|
| Ethylhexyl Triazone | 2,0 |
| Bis-Ethylhexyloxyphenol Methoxyphenyl Triazine | 1,0 |
| Butyl Methoxydibenzoylmethane | 3,0 |
| Ethylhexyl Methoxycinnamate BHT | 1,0 |
| Ethylhexyl Salicylate | 5,0 |
| Diisopropyl Sebacate | 8,0 |
| Dibutyl Adipate Behenyl Alcohol & Glyeryl Stearate & Glyceryl Stearate Citrate & | 3,0 |
| Sodium Dicocoylethylenediamine PEG-15 Sulfate | 6,0 |
| Cetearyl Alcohol | 1,0 |
| Cyclisches Siliconöl | 4,5 |
| Talkum Methylene Bis-Benzotriazolyl & | 2,0 |
| Tetramethylbutylphenol | 2,0 |

| **Phase C** | |
|---|---|
| Alcohol | 3,0 |
| Hydrolyzed Viola Tricolor Extract | 0,1 |
| Biotanning® | 0,25 |
| Sodium Lactate Methylsilanol & Salicylic Acid | 0,1 |
| RPF-Komplex* | 0,1 |
| Parfum | 0,8 |
| Phenoxyethanol | 0,6 |

Die Verfahrensweise entsprach der von Beispiel 1.

### Vergleichsbeispiel 3a Anti-Alterungscreme SPF 20

| **Phase A** | |
|---|---|
| Wasser | q.s. ad 100 |
| Caffeine | 0,55 |
| Sodium Polyacrylate | 0,6 |
| Propylene Glycol Diethylamino Hydroxybenzoyl Hexyl Benzoate & | 4,5 |
| Ethylhexyl Methoxycinnamate | 3,0 |

| **Phase B** | |
|---|---|
| Ethylhexyl Triazone | 3,0 |
| Bis-Ethylhexyloxyphenol Methoxyphenyl Triazine | 3,0 |
| Butyl Methoxydibenzoylmethane | 3,0 |
| Ethylhexyl Methoxycinnamate BHT | 1,0 |
| Ethylhexyl Salicylate | 7,0 |
| Diisopropyl Sebacate | 8,5 |
| Dibutyl Adipate Behenyl Alcohol & Glyeryl Stearate & Glyceryl Stearate Citrate & | 3,0 |
| Sodium Dicocoylethylenediamine PEG-15 Sulfate | 6,0 |
| Cetearyl Alcohol | 1,0 |
| Cyclisches Siliconöl | 4,5 |
| Talkum Methylene Bis-Benzotriazolyl & | 2,0 |
| Tetramethylbutylphenol | 3,0 |

| **Phase C** | |
|---|---|
| Alcohol | 3,0 |
| Hydrolyzed Viola Tricolor Extract | 0,1 |
| Biotanning® | 0,25 |
| Sodium Lactate Methylsilanol & Salicylic Acid | 0,1 |
| RPF-Komplex* | 0,15 |
| Parfum | 0,8 |
| Phenoxyethanol | 0,5 |

Die Verfahrensweise entsprach der von Beispiel 1.

### Beispiel 4

Es wurde eine Zusammensetzung gemäß Beispiel 2 hergestellt, mit Ausnahme dessen, dass anstelle von 0,5 % Disodium EDTA 0,6 Gew.-% eines Gemisches aus 0,5 % Disodium EDTA und 0,1 % Tyrosin eingesetzt wurde.

### Beispiel 5 in vivo-Test

Es wurden 20 Versuchsteilnehmer im Alter von 21-44 Jahren für einen in Test mit einer Creme gemäß Beispiel 1 und 1a ausgewählt. Die Versuchsbedingungen entsprachen denen, wie sie für den COLIPA-Test (Version 2003; s.a. Diffey et al., J. Soc. Cosmet.Chem., 40, 127-133 (1989)) festgelegt sind.

Es wurde jeweils eine Menge von 2 mg/cm² von beiden Cremes gemäß Vorschrift auf dem Rücken der Testpersonen aufgetragen und mit den minimalen erythemalen Dosen für ungeschützte und geschützte Haut (MEDu bzw. MEDp) bestrahlt.

Bei der Auswertung des Testes zeigten sich keine signifikanten Unterschiede, so dass davon auszugehen war, dass die Cremes gemäß Beispiel 1 und Beispiel 1a etwa gleichen Schutz boten. Dies unterstreicht die Überlegenheit der Erfindung, bei reduzierten UVA- und UVB-Filtern gleich gute Ergebnisse zu erreichen.

Ein zeitlich später durchgeführter in vivo-Test mit den Cremes der Beispiele 3 und 3a zeigte in der Auswertung das gleiche Ergebnis wie bei den Cremes der Beispiele 1 und 1a.

## Patentansprüche

1. Kosmetisches Sonnenschutzmittel, **gekennzeichnet durch** einen Gehalt an
11,5 bis 23 % organische UVA- und UVB-Sonnenschutzfilter, 0,4-1,2 % unter UV-Licht fluoreszierende Substanzen
und den Rest bis 100 % übliche kosmetische Trägerstoffe, Hilfsstoffe, Wirkstoffe oder Gemische davon,
wobei das kosmetische Mittel einen Sonnenschutzfaktor SPF im Bereich von SPF 12 bis SPF 35 hat und alle Prozentangaben auf das Gesamtgewicht des Mittels bezogen sind.

2. Sonnenschutzmittel nach Anspruch 1, **dadurch gekennzeichnet, dass** der Anteil der organischen UVB-Sonnenschutzfilter im Bereich von 6-13 % liegt und der Anteil der organischen UVA-Sonnenschutzfilter im Bereich von 5-11 % liegt.

3. Sonnenschutzmittel nach Anspruch 1, **dadurch gekennzeichnet, dass** das Mittel 0,6 bis 1,1 Gew.-% unter UV-Licht fluoreszierende Substanzen enthält.

4. Sonnenschutzmittel nach Anspruch 1, **dadurch gekennzeichnet, dass** das Mittel 0,5-1,2 % radikalfangende Substanzen enthält, insbesondere 0,5-1,0 Gew.-%.

5. Sonnenschutzmittel nach Anspruch 1, **dadurch gekennzeichnet, dass** die unter UV-Licht fluoreszierenden Substanzen weiße oder farblose Substanzen sind.

6. Sonnenschutzmittel nach Anspruch 1, **dadurch gekennzeichnet, dass** die unter UV-Licht fluoreszierenden Substanzen ausgewählt sind aus der Gruppe, bestehend aus Ethylendiamintetraessigsäure (EDTA), Dinatriumsalz von EDTA, Trinatriumsalz von EDTA, Tetranatriumsalz von EDTA, Tryptophan, Phenylalanin und Tyrosin.

7. Sonnenschutzmittel nach Anspruch 6, **dadurch gekennzeichnet, dass** die unter UV-Licht fluoreszierende Substanz das Dinatriumsalz von EDTA ist.

8. Sonnenschutzmittel nach Anspruch 1, **dadurch gekennzeichnet, dass** die UVA-Sonnenschutzfilter ausgewählt sind aus der Gruppe, bestehend aus Butyl Methoxydibenzoylmethane, Methylene Bis-Benzotriazolyl Tetramethylbutylphenol, Bis-Ethylhexyloxyphenol Methoxyphenyl Triazine, Diethylamino Hydroxybenzoyl Hexylbenzoate und Gemischen davon.

9. Sonnenschutzmittel nach Anspruch 1, **dadurch gekennzeichnet, dass** die UVB-Sonnenschutzfilter ausgewählt sind aus der Gruppe, bestehend aus Ethylhexyl Methoxycinnamate, Isoamyl-p-Methoxycinnamate, Ethylhexyl Triazone, Ethylhexyl Salicylate und Gemischen davon.

10. Sonnenschutzmittel nach Anspruch 1, **dadurch gekennzeichnet, dass** das Verhältnis zwischen UVB- und UVA-Sonnenschutzfiltern im Bereich von 1:1,1 bis 1,3 liegt.

11. Sonnenschutzmittel nach Anspruch 1, **dadurch gekennzeichnet, dass** es als Sonnenschutzfilter Diethylamino Hydroxybenzoyl Hexyl Benzoate & Ethylhexyl Methoxycinnamate, Ethylhexyl Triazone, Ethylhexyl Salicylate, Bis-Ethylhexyloxyphenol Methoxyphenyl Triazine, Butyl Methoxydibenzoylmethane, Methylene Bis-Benzotriazolyl & Tetramethylbutylphenol und Ethylhexyl Methoxycinnamate und wenigstens 0,6 % EDTA oder ein Salz von EDTA oder ein Gemisch davon enthält.

12. Verwendung von unter UV-Licht fluoreszierenden Verbindungen in einer Menge von 0,4 - 1,2 Gew.-% gemeinsam mit UV-Sonnenschutzfiltern in Sonnenschutzmitteln mit einem Sonnenschutzfaktor von SPF 12 bis SPF 35, wobei der Gehalt an UV-Filtern im Bereich von 11,5 bis 23 Gew.-% liegt, um Hautirritationen zu verringern oder zu vermeiden, die durch Konzentrationen von größer als 23 % an organischen UV-Sonnenschutzfiltern hervorgerufen werden.

13. Verwendung von unter UV-Licht fluoreszierenden Verbindungen in einer Menge von 0,4 - 1,2 Gew.-% gemeinsam mit UV-Sonnenschutzfiltern in Sonnenschutzmitteln mit einem Sonnenschutzfaktor von SPF 12 bis SPF 35, wobei der Gehalt an UV-Filtern im Bereich von 11,5 bis 23 Gew.-% liegt, um den Gehalt an UV-Filtern zu verringern, der ohne den Zusatz der fluoreszierenden Substanzen bei gleichem SPF erforderlich ist.

14. Verwendung nach einem der Ansprüche 12 und 13, wobei ein Gemisch von mehreren organischen UV-B-Filtern und einem oder mehreren organischen UV-A-Filtern vorliegt.

15. Verwendung nach Anspruch 14, wobei das Verhältnis zwischen UVB- und UVA-Sonnenschutzfiltern im Bereich von 1:1,1 bis 1,3 liegt.
